# EUROPEAN PATENT SPECIFICATION

(11) **EP 3 875 040 B1**
(45) Date of publication and mention of the grant of the patent: **28.05.2025**
(21) Application number: 19879588.2
(22) Date of filing: 01.11.2019
(51) Int. Cl.: A61B 17/072, A61B 17/00, A61B 90/00

(54) **ENSURING MECHANISM FOR ENSURING DEVICE MAY ONLY BE USED ONCE**
SICHERSTELLUNGSMECHANISMUS ZUR SICHERSTELLUNG, DASS EINE VORRICHTUNG NUR EINMAL VERWENDET WERDEN KANN
MÉCANISME D'ASSURANCE POUR GARANTIR QU'UN DISPOSITIF PEUT ÊTRE UNIQUEMENT UTILISÉ UNE FOIS

(30) Priority: 02.11.2018 CN 201811303644
(43) Date of publication of application: 08.09.2021
(73) Proprietor: Ezisurg (Suzhou) Medical Co., Ltd., Suzhou, Jiangsu 215163 (CN)
(72) Inventor: LIAO, Menghui, Shanghai 201318 (CN); WANG, Lei, Shanghai 201318 (CN); TANG, Chuangang, Shanghai 201318 (CN); YANG, Jun, Shanghai 201318 (CN); BAO, Minghui, Shanghai 201318 (CN); NIE, Honglin, Shanghai 201318 (CN)
(74) Representative: Mansfield, Peter Turquand
(86) International application number: PCT/CN2019/114937
(87) International publication number: WO 2020/088629

(56) References cited:
- CN-A- 102 885 641
- CN-A- 103 767 751
- CN-A- 105 682 570
- CN-U- 204 708 912
- US-A1- 2005 222 616
- US-A1- 2013 334 284

## Description

### Technical Field

The present disclosure relates to the field of medical instruments, and in particular, to a safety mechanism for ensuring disposable use of a staple cartridge in a stapler.

### Background Art

The stapler is a main medical instrument that is medically used in place of conventional manual suturing, and has the advantages of convenience in use, rapid suture, dense suture, moderate tightness, and fewer side effects and surgical complications.

Currently, schemes used in the stapler for times-limiting are as follows: a scheme used for times-limiting adopted by Johnson & Johnson is a manner of pressing a cutter by a spring sheet, wherein the spring sheet is connected to a staple cartridge base by means of electric welding. This technical solution has an obvious defect that if the elastic force of the spring sheet fails or a welding spot falls off, this times-limiting mechanism is vulnerable to failures, resulting in a failure of instrument or even death of patient. Covidien company adopts a times-limiting mechanism placed in a staple casing inner tube, wherein a times- limiting clamping piece is connected to a sleeve, wherein the sleeve is connected to a firing drive of the stapler, an initial state refers to that the times- limiting clamping piece abuts against a guide rail in the staple casing inner tube under the action of a spring, when the firing drive drives the times-limiting clamping structure to move forwards, the times-limiting clamping piece is divorced from the rail, and when the firing drive is reset and performs firing again, the times-limiting clamping piece then abuts against a boss in the staple casing inner tube. The defect of this technical solution is that the strength of a plastic inner tube and the times-limiting clamping piece cannot be very high, and if a relatively great human force is encountered, there is a risk of damaging them, and the times-limiting mechanism fails.

US 2005/0222616 (US Surgical) describes a surgical instrument that includes a replaceable staple cartridge, a knife, a staple anvil and a central track on the cartridge base, and it includes a safety lockout for the cartridge, which engages a hook on the knife; once the stapler has been partially or completely fired and is unclamped, this safety lockout prevents the stapler from being re-clamped until the staple cartridge has been removed and replaced. The instrument also includes a sliding block as part of a clamp latch and safety interlock mechanism to prevent the instrument opening during the firing stroke.

### Summary

The invention is defined in independent claim 1. Certain optional features of the invention are defined in the dependent claims. In view of the shortcomings in the prior art, the technical problem to be solved in the present disclosure is to provide a safety mechanism for ensuring disposable use of a staple cartridge in a stapler, to ensure that the staple cartridge in the stapler can only be used once in surgery, and to guarantee the patients' safety.

According to one aspect of the present disclosure, a safety mechanism for ensuring disposable use of a staple cartridge in a stapler is provided, as defined in claim 1.

As a further option, a third guide structure, separated from the first guide structure, is further provided on the cutter.

As a further option, when the staple cartridge is assembled in the staple cartridge base, the sliding block is positioned between the staple cartridge base and the staple cartridge, and located in an initial position, and at a distal end of the cutter, and the first guide structure is in contact with the second guide structure.

As a further option, a bias structure is provided at a proximal end of the staple anvil, the bias structure is corresponding to the bias track of the staple cartridge base, and the bias structure guides the cutter to enter the bias track of the staple cartridge base. The bias structure is, for example, a bias boss.

As a further option, the cutter is fixedly connected at a distal end of a cutter bar assembly.

As a further option, the first guide structure is a guide protrusion; and the second guide structure is a guide surface.

As a further option, the guide surface is an inclined surface inclined in a direction from outside to a central axis of the staple cartridge base.

As a further option, at the initial position of the sliding block, the guide protrusion of the cutter is in contact with a proximal end of the guide surface of the sliding block. When the cutter continues moving forward, the guide protrusion of the cutter enters, under the guiding effect of the guide surface of the sliding block, the central track of the staple cartridge base.

As a further option, when the sliding block is not at the initial position, the cutter enters the bias track of the staple cartridge base due to restriction of the bias boss of the staple anvil, and when the cutter continues moving forwards, a locking projection enters the blocking groove of the staple cartridge base, and is restricted from moving forwards.

As a further option, the third guide structure is a locking projection.

As a further option, the guide protrusion and the locking projection are provided at two sides of the cutter in a horizontal direction, respectively.

According to another aspect of the present disclosure, the safety mechanism defined above forms part of a surgical instrument and the surgical instrument is a stapler.

As a further option, the stapler includes, but is not limited to, disposable stapler, reusable stapler, electric stapler, etc.

The safety mechanism for ensuring disposable use of a staple cartridge in a stapler in the present disclosure has a simple and effective structure, and once the safety mechanism takes effect, it is difficult to destroy it by human force, thus ensuring the patients' safety of using the instrument.

### Brief Description of Drawings

In order to more clearly illustrate technical solutions of the present disclosure, accompanying drawings which need to be used for description of the embodiments or the prior art will be introduced briefly below, and it will be apparent that the accompanying drawings in the description below merely show some embodiments of the present disclosure, and those ordinarily skilled in the art still could obtain other accompanying drawings in light of these accompanying drawings, without any creative efforts.
FIG. 1 is a schematic view of states of various parts when a sliding block 3 is at an initial position;
FIG. 2 is a schematic view of a state when a cutter 4 is guided by the sliding block 3 to enter a central track;
FIG. 3 is a schematic view of a state when the sliding block 3 is not at the initial position;
FIG. 4 is a schematic view of a guide surface and a locking projection of the cutter 4;
FIG. 5 is a structural schematic view of a guide surface of the sliding block 3;
FIG. 6 is a schematic view of a bias boss of a staple anvil 8; and
FIG. 7 is a structural schematic view of a bias track and a blocking groove of the staple cartridge base 2.

### Detailed Description of Embodiments

Technical solutions in the embodiments of the present disclosure will be described clearly and completely below in connection with the accompanying drawings of the description, and it will be apparent that the embodiments described are only some but not all embodiments of the present disclosure. All other embodiments obtained by those ordinarily skilled in the art based on the embodiments of the present disclosure without any creative efforts shall fall within the scope of protection of the present disclosure, which is defined by the claims.

The term "exemplary" is used herein to mean "serving as an example, instance, or illustration". Any implementation described herein as "exemplary" is not necessarily to be construed as being better than or superior to other implementations. Moreover, in the text, "proximal end", "proximal", and "rear" refer to an end proximate to an operator, and "distal end", "distal", and "front" refer to an end away from the operator.

For the sake of simplicity, a stapler is taken as an example to illustrate the instrument of the present disclosure. Those skilled in the art could understand that a safety mechanism for ensuring disposable use of an instrument in the present disclosure is not limited to a stapler. In other products such as closures and electric scalpels, the technology of the present disclosure may also be adopted.

For the safety mechanism for ensuring disposable use of a staple cartridge in a stapler in the present disclosure, the stapler includes an actuator located at a distal end, a controller located at a proximal end, and an extension tube located between the actuator and the controller. The extension tube is movably connected to a distal end of the controller, and a distal end of the extension tube is pivotably connected to a proximal end of the actuator, preferably by one or more hinges. The extension tube constitutes one connecting channel for transferring an action made by the controller to the actuator. The actuator includes a staple anvil assembly and a staple cartridge assembly that can clamp tissues therebetween.

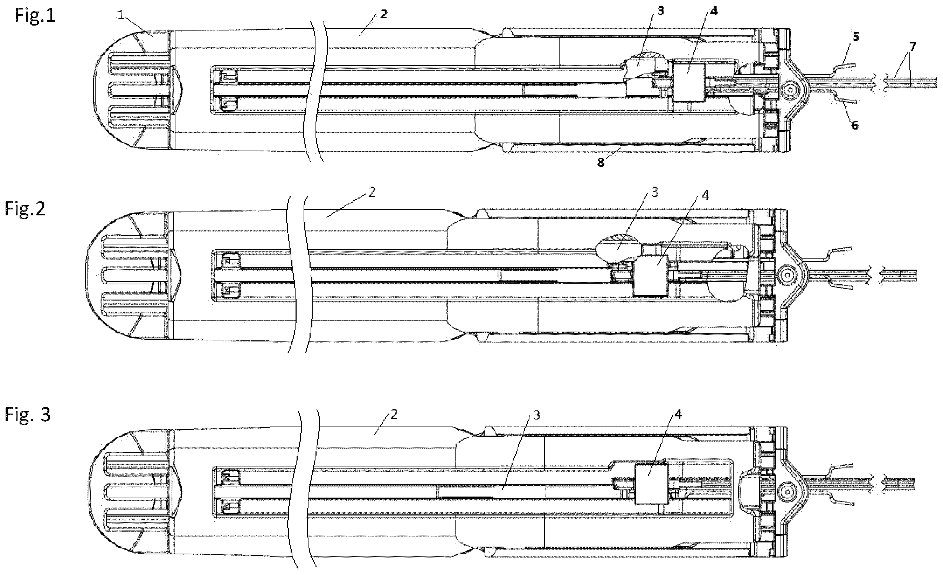

Specifically, the safety mechanism for ensuring disposable use of a staple cartridge in a stapler according to the present disclosure includes a staple cartridge 1, a staple cartridge base 2, a sliding block 3, a cutter 4, protective plates 5 and 6, a cutter bar assembly 7, and a staple anvil 8, wherein the staple cartridge base 2 is centrally provided with a central track, and the sliding block is provided on the staple cartridge base; with reference to FIG. 1, the cutter 4 is fixedly connected at a distal end of the cutter bar assembly 7.

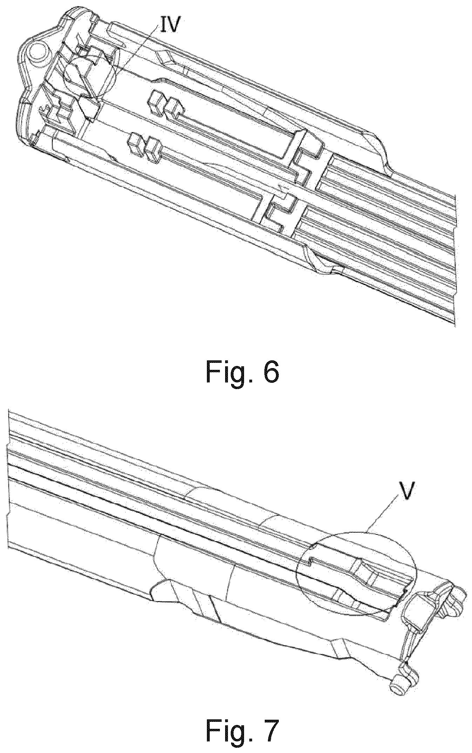

With reference to FIG. 7, a bias track is provided at a proximal end of the staple cartridge base 2, marked as V in FIG. 7, and the bias track is in communication with a proximal end of the central track of the staple cartridge base 2. A blocking groove is provided at a distal end of the bias track, marked as V in FIG. 7.

With reference to FIG. 6, a bias boss is provided at a proximal end of the staple anvil 8, marked as IV in FIG. 6, and the bias boss is corresponding to the bias track of the staple cartridge base 2.

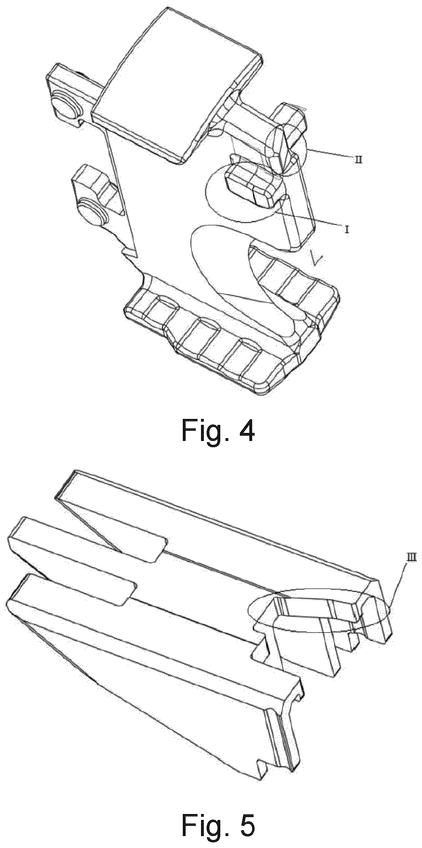

With reference to FIG. 4, a guide protrusion is provided on the cutter 4, marked as I in FIG. 4; and the cutter 4 is further provided with a locking projection, marked as II in FIG. 4.

With reference to FIG. 5, the sliding block 3 is provided with a guide surface corresponding to the guide protrusion of the cutter 4, marked as III in FIG. 5, and the guide surface is an inclined surface inclined in a direction from outside to a central axis of the staple cartridge base 2, so that the guide protrusion can be guided from a biased position to a central position where the guide protrusion may move forwards, so that the guide protrusion may enter the central track.

FIG. 1 is a structural schematic view of an embodiment of the present disclosure. The state in the figure is that the sliding block 3 is at an initial position. The sliding block 3 is assembled at the proximal end of the staple cartridge 1 before delivery, and when the staple cartridge 1 is assembled into the staple cartridge base 2, the sliding block 3 is positioned between the staple cartridge base and the staple cartridge, and located at the initial position, at the distal end of the cutter 4. At this time, the cutter bar assembly 7 is in contact with the bias boss (marked as IV in FIG. 6) at the proximal end of the staple anvil 8, so that the cutter 4 is biased into the bias track of the staple cartridge base 2 (marked as V in FIG. 7), at this time, the guide protrusion (marked as I in FIG. 4) of the cutter 4 comes into contact with a proximal end of the guide surface (marked as III in FIG. 5) of the sliding block 3; when the cutter 4 and the cutter bar assembly 7 continue moving forward, the guide protrusion (marked as I in FIG. 4) of the cutter 4 enters the central track of the staple cartridge base under the guiding effect of the guide surface (marked as III in FIG. 5) of the sliding block 3, as in FIG. 2, and at this time the cutter 4 may continue moving forwards.

When the sliding block 3 is not at the initial position, as in FIG. 3, the cutter 4 enters the bias track of the staple cartridge base 2 due to restriction of the bias boss of the staple anvil 8, and in this case, when the cutter 4 continues moving forwards without the restriction of the guide surface of the sliding block 3, the locking projection (marked as II in FIG. 4) of the cutter 4 enters the blocking groove (marked as V in FIG. 7) of the staple cartridge base 2, and is restricted from moving forwards.

Specifically, the guide protrusion and the locking projection on the cutter are provided at two sides of the cutter in a horizontal direction (i.e. a direction perpendicular to a central axis of the stapler), respectively.

The safety mechanism for ensuring disposable use of a staple cartridge in a stapler in the present disclosure has a simple and effective structure, and once the safety mechanism takes effect, it is difficult to destroy it by human force, thus ensuring the patients' safety in using the instrument.

The above-mentioned are preferred embodiments of the present disclosure, and it should be indicated that those ordinarily skilled in the art still could make improvements and modifications, without departing from the principle of the present disclosure, and all of these improvements and modifications also should be considered as within the scope of protection of the present disclosure, which is defined by the claims.

## Claims

1. A safety mechanism for ensuring disposable use of a staple cartridge (1) in a stapler, comprising: the staple cartridge (1), a staple cartridge base (2), a sliding block (3), a cutter (4), and a staple anvil (8), wherein the staple cartridge base (2) is centrally provided with a central track, a proximal end of the staple cartridge base (2) is provided with a bias track (V), the bias track (V) is in communication with a proximal end of the central track, a distal end of the bias track (V) is provided with a blocking groove, and the sliding block is assembled at a proximal end of the staple cartridge; a first guide structure (I) is provided on the cutter (4); **characterised in that** the sliding block (3) is provided with a second guide structure (III) corresponding to the first guide structure (I) of the cutter (4), and the second guide structure (III) is capable of guiding the first guide structure (I) to a central position from a biased position, so that the cutter (4) enters the central track.

2. The safety mechanism according to claim 1, wherein a third guide structure (II) provided separated from the first guide structure (I) is further provided on the cutter (4).

3. The safety mechanism according to claim 2, wherein when the staple cartridge (1) is assembled in the staple cartridge base (2), the sliding block (3) is positioned between the staple cartridge base (2) and the staple cartridge (1), and located in an initial position, and at a distal end of the cutter (4), and the first guide structure (I) is in contact with the second guide structure (III).

4. The safety mechanism according to claim 3, wherein a bias structure (IV) is provided at a proximal end of the staple anvil (8), the bias structure (IV) is corresponding to the bias track (V) of the staple cartridge base (2), and the bias structure (IV) guides the cutter (4) to enter the bias track (V) of the staple cartridge base (2).

5. The safety mechanism according to claim 4, wherein the bias structure is a bias boss (IV).

6. The safety mechanism according to any one of claims 1-5, wherein the cutter (4) is fixedly connected at a distal end of a cutter bar assembly (7).

7. The safety mechanism according to claim 4 or 5, wherein the first guide structure (I) is a guide protrusion; and the second guide structure (III) is a guide surface, and the third guide structure (II) is a locking projection.

8. The safety mechanism according to claim 7, wherein the guide surface (III) is an inclined surface inclined in a direction from outside to a central axis of the staple cartridge base (2).

9. The safety mechanism according to claim 7, wherein the guide protrusion (I) and the locking projection (II) are provided at two sides of the cutter (4) in a horizontal direction, respectively.

10. The safety mechanism according to claim 9, wherein at the initial position of the sliding block (3), the guide protrusion (I) of the cutter (4) is in contact with a proximal end of the guide surface (III) of the sliding block (3); and when the cutter (4) continues moving forward, the guide protrusion (I) enters, under a guiding effect of the guide surface (III) of the sliding block (3), the central track of the staple cartridge base (2).

11. The safety mechanism according to claim 9, wherein when the sliding block (3) is not at the initial position, the cutter (4) enters the bias track (V) of the staple cartridge base (2) due to restriction of the bias structure (IV) of the staple anvil (8), and when the cutter (4) continues moving forwards, the third guide structure (II) enters the blocking groove of the staple cartridge base (2), and is restricted from moving forwards.

12. Surgical instrument for ensuring disposable use of a staple cartridge, and comprising the safety mechanism as claimed in any one of the preceding claims.

## Patentansprüche

1. Sicherheitsmechanismus zur Sicherstellung einer Einwegverwendung eines Klammermagazins (1) in einer Klammervorrichtung, umfassend: das Klammermagazin (1), eine Klammermagazinbasis (2), einen Gleitblock (3), eine Schneidvorrichtung (4) und einen Klammeramboss (8), wobei die Klammermagazinbasis (2) zentral mit einer zentralen Bahn versehen ist, ein proximales Ende der Klammermagazinbasis (2) mit einer Vorspannungsbahn (V) versehen ist, die Vorspannungsbahn (V) in Verbindung mit einem proximalen Ende der zentralen Bahn steht, ein distales Ende der Vorspannungsbahn (V) mit einer Blockierrille versehen ist und der Gleitblock an einem proximalen Ende des Klammermagazins montiert ist; wobei eine erste Führungsstruktur (I) an der Schneidvorrichtung (4) vorgesehen ist; **dadurch gekennzeichnet, dass**
der Gleitblock (3) mit einer zweiten Führungsstruktur (III) versehen ist, die der ersten Führungsstruktur (I) der Schneidvorrichtung (4) entspricht, und die zweite Führungsstruktur (III) dazu in der Lage ist, die erste Führungsstruktur (I) in eine zentrale Position aus einer vorgespannten Position zu führen, so dass die Schneidvorrichtung (4) in die zentrale Bahn eintritt.

2. Sicherheitsmechanismus nach Anspruch 1, wobei eine dritte Führungsstruktur (II), die getrennt von der ersten Führungsstruktur (I) vorgesehen ist, ferner an der Schneidvorrichtung (4) vorgesehen ist.

3. Sicherheitsmechanismus nach Anspruch 2, wobei, wenn das Klammermagazin (1) in der Klammermagazinbasis (2) montiert ist, der Gleitblock (3) zwischen der Klammermagazinbasis (2) und dem Klammermagazin (1) positioniert ist und sich in einer Anfangsposition und an einem distalen Ende der Schneidvorrichtung (4) befindet, und die erste Führungsstruktur (I) in Kontakt mit der zweiten Führungsstruktur (III) steht.

4. Sicherheitsmechanismus nach Anspruch 3, wobei eine Vorspannungsstruktur (IV) an einem proximalen Ende des Klammerambosses (8) vorgesehen ist, die Vorspannungsstruktur (IV) der Vorspannungsbahn (V) der Klammermagazinbasis (2) entspricht und die Vorspannungsstruktur (IV) die Schneidvorrichtung (4) führt, um in die Vorspannungsbahn (V) der Klammermagazinbasis (2) einzutreten.

5. Sicherheitsmechanismus nach Anspruch 4, wobei die Vorspannungsstruktur ein Vorspannungsansatz (IV) ist.

6. Sicherheitsmechanismus nach einem der Ansprüche 1-5, wobei die Schneidvorrichtung (4) fest an einem distalen Ende einer Schneidleistenanordnung (7) angebracht ist.

7. Sicherheitsmechanismus nach Anspruch 4 oder 5, wobei die erste Führungsstruktur (I) ein Führungsvorsprung ist; und die zweite Führungsstruktur (III) eine Führungsfläche ist und die dritte Führungsstruktur (II) eine Arretiernase ist.

8. Sicherheitsmechanismus nach Anspruch 7, wobei die Führungsfläche (III) eine geneigte Fläche ist, die in einer Richtung von der Außenseite zu einer zentralen Achse der Klammermagazinbasis (2) geneigt ist.

9. Sicherheitsmechanismus nach Anspruch 7, wobei der Führungsvorsprung (I) und die Arretiernase (II) jeweils an zwei Seiten der Schneidvorrichtung (4) in einer horizontalen Richtung vorgesehen sind.

10. Sicherheitsmechanismus nach Anspruch 9, wobei in der Anfangsposition des Gleitblocks (3) der Führungsvorsprung (I) der Schneidvorrichtung (4) in Kontakt mit einem proximalen Ende der Führungsfläche (III) des Gleitblocks (3) steht; und wenn die Schneidvorrichtung (4) fortfährt, sich nach vorne zu bewegen, der Führungsvorsprung (I) unter einer Führungswirkung der Führungsfläche (III) des Gleitblocks (3) in die zentrale Bahn der Klammermagazinbasis (2) eintritt.

11. Sicherheitsmechanismus nach Anspruch 9, wobei, wenn der Gleitblock (3) nicht in der Anfangsposition ist, die Schneidvorrichtung (4) in die Vorspannungsbahn (V) der Klammermagazinbasis (2) aufgrund der Begrenzung der Vorspannungsstruktur (IV) des Klammerambosses (8) eintritt, und wenn die Schneidvorrichtung (4) fortfährt, sich nach vorne zu bewegen, die dritte Führungsstruktur (II) in die Blockierrille der Klammermagazinbasis (2) eintritt und daran gehindert wird, sich nach vorne zu bewegen.

12. Chirurgische Instrument zur Sicherstellung einer Einwegverwendung eines Klammermagazins und umfassend den Sicherheitsmechanismus nach einem der vorhergehenden Ansprüche.

## Revendications

1. Mécanisme de sécurité destiné à assurer l'utilisation à usage unique d'une cartouche d'agrafes (1) dans une agrafeuse, comprenant : la cartouche d'agrafes (1), une base de cartouche d'agrafes (2), un bloc coulissant (3), un élément de coupe (4), et une enclume d'agrafe (8), dans lequel la base de cartouche d'agrafes (2) est pourvue de manière centrale d'une piste centrale, une extrémité proximale de la base de cartouche d'agrafes (2) est pourvue d'une piste de sollicitation (V), la piste de sollicitation (V) est en communication avec une extrémité proximale de la piste centrale, une extrémité distale de la piste de sollicitation (V) est pourvue d'une rainure de blocage, et le bloc coulissant est assemblé au niveau d'une extrémité proximale de la cartouche d'agrafes ; une première structure de guidage (I) est fournie sur l'élément de coupe (4) ; **caractérisé en ce que** le bloc coulissant (3) est pourvu d'une deuxième structure de guidage (III) correspondant à la première structure de guidage (I) de l'élément de coupe (4), et la deuxième structure de guidage (III) est capable de guider la première structure de guidage (I) vers une position centrale à partir d'une position sollicitée, de sorte que l'élément de coupe (4) pénètre dans la voie centrale.

2. Mécanisme de sécurité selon la revendication 1, dans lequel une troisième structure de guidage (II) fournie séparée de la première structure de guidage (I) est fournie en outre sur l'élément de coupe (4).

3. Mécanisme de sécurité selon la revendication 2, dans lequel lorsque la cartouche d'agrafes (1) est assemblée dans la base de cartouche d'agrafes (2), le bloc coulissant (3) est positionné entre la base de cartouche d'agrafes (2) et la cartouche d'agrafes (1), et situé dans une position initiale, et au niveau d'une extrémité distale de l'élément de coupe (4), et la première structure de guidage (I) est en contact avec la deuxième structure de guidage (III).

4. Mécanisme de sécurité selon la revendication 3, dans lequel une structure de sollicitation (IV) est fournie au niveau d'une extrémité proximale de l'enclume d'agrafe (8), la structure de sollicitation (IV) correspond à la piste de sollicitation (V) de la base de cartouche d'agrafes (2), et la structure de sollicitation (IV) guide l'élément de coupe (4) pour qu'il pénètre dans la piste de sollicitation (V) de la base de cartouche d'agrafes (2).

5. Mécanisme de sécurité selon la revendication 4, dans lequel la structure de sollicitation est un bossage de sollicitation (IV).

6. Mécanisme de sécurité selon l'une quelconque des revendications 1 à 5, dans lequel l'élément de coupe (4) est relié de manière fixe à une extrémité distale d'un ensemble de barre d'élément de coupe (7).

7. Mécanisme de sécurité selon la revendication 4 ou 5, dans lequel la première structure de guidage (I) est une saillie de guidage ; et la deuxième structure de guidage (III) est une surface de guidage, et la troisième structure de guidage (II) est une saillie de verrouillage.

8. Mécanisme de sécurité selon la revendication 7, dans lequel la surface de guidage (III) est une surface inclinée, inclinée dans une direction allant de l'extérieur vers un axe central de la base de cartouche d'agrafes (2).

9. Mécanisme de sécurité selon la revendication 7, dans lequel la saillie de guidage (I) et la saillie de verrouillage (II) sont fournies au niveau de deux côtés de l'élément de coupe (4) dans une direction horizontale, respectivement.

10. Mécanisme de sécurité selon la revendication 9, dans lequel dans la position initiale du bloc coulissant (3), la saillie de guidage (I) de l'élément de coupe (4) est en contact avec une extrémité proximale de la surface de guidage (III) du bloc coulissant (3) ; et lorsque l'élément de coupe (4) continue à se déplacer vers l'avant, la saillie de guidage (I) pénètre, sous un effet de guidage de la surface de guidage (III) du bloc coulissant (3), dans la piste centrale de la base de cartouche d'agrafes (2).

11. Mécanisme de sécurité selon la revendication 9, dans lequel lorsque le bloc coulissant (3) n'est pas dans la position initiale, l'élément de coupe (4) pénètre dans la piste de sollicitation (V) de la base de cartouche d'agrafes (2) en raison de la restriction de la structure de sollicitation (IV) de l'enclume d'agrafe (8), et lorsque l'élément de coupe (4) continue à se déplacer vers l'avant, la troisième structure de guidage (II) pénètre dans la rainure de blocage de la base de cartouche d'agrafes (2), et est empêchée de se déplacer vers l'avant.

12. Instrument chirurgical destiné à assurer l'utilisation à usage unique d'une cartouche d'agrafes, et comprenant le mécanisme de sécurité selon l'une quelconque des revendications précédentes.
